# FASCICULE DE BREVET EUROPEEN

(11) **EP 1 035 836 B1**
(45) Date de publication et mention de la délivrance du brevet: **03.06.2009**
(21) Numéro de dépôt: 98958288.7
(22) Date de dépôt: 01.12.1998
(51) Int. Cl.: A61K 9/127, C12N 9/96, A61K 38/48

(54) **COMPOSITION CONTENANT UNE ENZYME ET UN AGENT STABILISANT LADITE ENZYME ET PROCEDE POUR AMELIORER LA STABILITE D'UNE ENZYME ET EVITER SA DEGRADATION OU POUR PROTEGER ET/OU IMMOBILISER UNE ENZYME**
ZUSAMMENSETZUNG ENTHALTEND EIN ENZYM UND EINEN STABILISATOR FÜR DIESES ENZYM UND PROZESS UM DIE STABILITÄT DES ENZYMS ZU VERBESSERN, SEINEN ABBAU ZU VERHINDERN, UM ES ZU SCHÜTZEN UND/ODER UM ES ZU IMMOBILISIEREN
COMPOSITION COMPRISING AN ENZYM, A STABILIZER FOR THE ENZYM AND A PROCESS FOR AMELIORATING THE STABILITY OF THE ENZYME, TO PREVENT ITS DEGRADATION, TO PROTECT IT AND/OR TO IMMOBILISE IT

(30) Priorité: 01.12.1997 FR 9715073; 27.03.1998 FR 9803807
(43) Date de publication de la demande: 20.09.2000
(73) Titulaire: CAPSULIS, 33600 Pessac (FR)
(72) Inventeur: DEGERT, Corinne, F-33160 Saint Médard en Jalles (FR); LAVERSANNE, René, F-33600 Pessac (FR); ROUX, Didier, F-33700 Mérignac (FR); UGAZIO, Stéphane, F-33000 Bordeaux (FR)
(74) Mandataire: Giraud, Françoise
(86) Numéro de dépôt international: PCT/FR1998/002579
(87) Numéro de publication internationale: WO 1999/027907

(56) Documents cités:
- EP-A- 0 688 563
- WO-A-93/19735
- WO-A-95/18601
- WO-A-95/33448
- WO-A-96/31194
- FR-A- 2 221 122
- FR-A- 2 735 658
- US-A- 4 235 871
- US-A- 5 139 803
- US-A- 5 279 834

## Description

La présente invention concerne une composition contenant une enzyme et un agent destiné à éviter la dégradation de cette enzyme.

Elle concerne également un procédé pour améliorer la stabilité d'une enzyme et éviter sa dégradation ainsi qu'un procédé pour protéger et/ou immobiliser une enzyme.

Que ce soit en cosmétique, en pharmacie, en détergence ou encore en agroalimentaire, la fonctionnalité d'un produit est généralement due à la présence d'une molécule dite matière active ou agent actif. A titre d'exemple, on peut citer les vitamines, utilisées en agro-alimentaire et en pharmacie, les enzymes utilisées en détergence, les organo-phosphorés utilisés comme insecticides, ou encore les arômes et parfums utilisés dans l'hygiène, l'agro-alimentaire ou la cosmétique.

L'une des caractéristiques essentielles d'un produit industriel ou pharmaceutique, outre son activité et son efficacité, est sa stabilité, de manière à obtenir un produit ayant une durée de vie suffisante. Malheureusement, de nombreuses matières actives sont des molécules particulièrement fragiles, dont la dégradation sous l'effet des contraintes d'environnement est trop rapide pour obtenir la durée de vie souhaitée du produit la contenant. C'est le cas de certaines vitamines comme les vitamines C, A ou E, de beaucoup d'enzymes comme par exemple les protéases, et plus généralement de beaucoup de protéines et de molécules biologiques, ou encore dans le domaine des insecticides, du malathion et des pyréthrinoïdes, et, d'une manière plus générale, des molécules réductrices ou oxydantes.

De nombreuses stratégies ont été élaborées pour éviter la dégradation de ces molécules actives fragiles. Ces stratégies dépendent de la nature de la réaction provoquant la dégradation. Les réactions les plus souvent incriminées sont soit l'hydrolyse soit l'oxydoréduction. D'autres réactions plus spécifiques ont aussi lieu, comme l'autoprotéolyse dans le cas des protéases.

Dans le cas où l'eau est une cause directe ou indirecte de la dégradation, une solution simple consiste à éviter le contact de la molécule active avec un milieu aqueux. C'est le cas par exemple d'insecticides dissous dans un solvant organique, et mis en oeuvre en aérosol. Malheureusement cette possibilité n'existe pas toujours, comme par exemple pour la cosmétique ou l'agro-alimentaire, qui, pour des raisons évidentes, ne peuvent utiliser les solvants organiques. De plus, la tendance actuelle, pour des raisons écologiques et de santé publique, est à la suppression de l'utilisation de solvants organiques dans toutes les branches de l'industrie.

Lorsque l'eau n'est qu'une cause indirecte, par exemple par effet d'oxydation par l'oxygène dissous, on peut travailler avec une eau dégazée. D'une manière plus générale, on peut travailler en atmosphère inerte, à la fois pour la fabrication et la conservation du produit final. Cette solution est souvent employée en agro-alimentaire, où les produits liquides enrichis en vitamines sont conditionnés soit sous vide d'air, soit sous atmosphère inerte. Malheureusement cette méthode ne permet de limiter la dégradation que jusqu'à l'ouverture du contenant. Elle n'est donc pas applicable dans le cas de produits nécessitant une longue durée de vie après ouverture.

Les enzymes sont des protéines qui catalysent de manière très spécifique des réactions chimiques. Elles sont très utilisées industriellement, par exemple dans les lessives pour dégrader les protéines (protéases), les lipides (lipases) ou les résidus amylacés (amylases), facilitant ainsi le nettoyage. Ces protéines sont en général instables en solution, et sont donc surtout utilisées dans les lessives en poudre. Leur utilisation en lessive liquide (pour le linge ou la vaisselle) est très limitée par leur instabilité à long terme.

De même, une tendance actuelle est à l'utilisation d'enzymes en cosmétique, par exemple des protéases pour aider à la desquamation de la peau, et donc à son renouvellement. Là encore l'instabilité des enzymes empêche leur utilisation simple.

L'immobilisation d'enzymes est un sujet très développé, au moins en recherche, les applications industrielles n'étant pas encore très nombreuses. Les travaux sur l'encapsulation d'enzymes font partie de ceux du domaine de la bioencapsulation qui comprend aussi l'encapsulation d'organismes "vivants" (levures, bactéries...). En général il est fait appel à des polymères qui forment une matrice immobilisant l'enzyme.

Dans ces travaux, il s'agit généralement d'immobiliser l'enzyme (ou la levure) pour l'avoir sous forme facilement manipulable, comme par exemple des microbilles que l'on peut extraire du milieu de réaction, après usage, par simple filtration ou tamisage suivant leur taille. Par conséquent la forme encapsulée de l'enzyme doit garder son activité. Le matériau d'encapsulation doit être suffisamment poreux pour laisser diffuser le substrat et les produits de réaction. Une revue de ces développements peut être trouvée dans "Bioencapsualtion in biotechnology, Biomat., Art. Cells & Immob. Biotech., 21, 291-297 (1993)".

Les principales réactions conduisant à la perte d'activité d'une enzyme sont la dégradation chimique, la perte de configuration spatiale ou l'agrégation de plusieurs enzymes. Les méthodes typiquement utilisées pour éviter ou limiter les excès de ces dégradations sont la modification chimique de la molécule et l'immobilisation.

La protection d'enzymes vis à vis de leur dénaturation par encapsulation est moins courante. En effet, les matrices polymères utilisées dans l'immobilisation laissant diffuser les substrats, elles sont trop poreuses pour réellement protéger l'enzyme. Les principales réactions de dégradation, sont celles touchant les protéines. Il s'agit, en particulier, d'hydrolyses spécifiques des liaisons entre acides aminés et de réactions conduisant à une dénaturation par changement de conformation n'impliquant pas de liaisons covalentes, mais entrainant une perte d'activité de l'enzyme, par modification de l'accessibilité du site catalyseur par exemple. Ainsi dans l'article "Prolonged retention of cross-linked trypsin in calcium alginate microspheres, J. Microencapsulation, 14, 51-61 (1997)", il est montré qu'il est nécessaire de réticuler préalablement l'enzyme par du glutaraldéhyde pour assurer sa protection, ce qui induit une perte de 50 % de son activité.

Dans le cas des enzymes, par exemple dans les lessives liquides ou dans les crèmes cosmétiques, il n'est pas possible d'utiliser un solvant non aqueux, à la fois pour des raisons de sécurité et de coût. L'introduction d'enzymes dans ce type de produit se heurte donc à des difficultés réelles. Une solution, parfois adoptée en cosmétique consiste à utiliser un emballage sophistiqué, constitué de deux réservoirs indépendants, l'un contenant la molécule active, l'autre contenant le reste de la préparation. Le mélange est effectué extemporanément au moment de l'utilisation du produit, grâce à un système de double pompe doseuse sur le flacon. Cette solution est assez onéreuse, et peu commode d'utilisation. Cette solution a aussi été mise en oeuvre dans le cas de la cosmétique pour proposer des produits de beauté contenant des vitamines.

Une autre méthode consiste aussi à séparer l'actif de son milieu, mais de manière microscopique en le microencapsulant dans des microsphères de polymère, ou en l'enrobant, par exemple par une technique d'enrobage en lit fluidisé, dans une matrice polymère. Cette technique peut s'avérer intéressante, en particulier pour des produits destinés à être incorporés dans des formes sèches. Elle présente l'inconvénient de nécessiter la rupture de la coque ou de l'enrobage polymère pour libérer l'actif. Elle est donc peu adaptée à des produits cosmétiques
où la libération de l'actif doit se faire spontanément au moment de l'application du produit, ou à des produits alimentaires, qui doivent libérer leur actif en bouche.

Lorsque l'instabilité de la matière active est modérée, l'utilisation de molécules protectrices peut s'avérer intéressante. C'est le cas des agents anti-oxydants largement utilisés dans les crèmes cosmétiques et les produits alimentaires. Ils sont en général simplement additionnés à la préparation, mais du fait de la dilution il est nécessaire d'en ajouter une quantité plus importante que nécessaire pour obtenir un effet. D'autre part, comme beaucoup d'additifs, leur usage tend à être de plus en plus réglementé, et les doses autorisées diminuées.

Il est connu d'encapsuler des principes actifs dans des vésicules à base de tensioactifs.

Ces vésicules présentent généralement une ou plusieurs bicouches. On parlera de vésicules unilamellaires lorsqu'elles sont constituées d'un coeur aqueux entouré d'une bicouche de tensioactifs et de vésicules paucilamellaires ou multilamellaires lorsqu'elles présentent plusieurs bicouches. Parmi les vésicules multilamellaires, on distingue celles que l'on désignera ci-après par "vésicules multilamellaires ou MLV de type classique" et des vésicules de structure bien particulière que l'on désignera ci-après par "vésicules multilamellaires à structure en oignon". Ces deux types de vésicules multilamellaires se distinguent par trois différences fondamentales :

### 1°. Leur procédé d'obtention

Le procédé d'obtention des MLV classiques fait généralement appel à un mélange préliminaire en milieu solvant organique des lipides et autres composants constituant l'enveloppe desdites MLV, puis à l'évaporation du solvant pour obtenir un film sec. Les vésicules sont ensuite obtenues par réhydratation de ce film de lipides par une solution aqueuse contenant l'agent actif à encapsuler.

Les vésicules multilamellaires à structure en oignon sont, quant à elles, obtenues par cisaillement d'une phase lamellaire cristal-liquide comprenant l'actif à encapsuler.

### 2°. Leur structure

Du fait de leur mode de préparation, les MLV classiques sont des agrégats de feuillets multilamellaires de type lipidique rassemblés au sein d'une membrane lipidique approximativement sphérique. Cette structure apparaît très clairement dans le cliché donné dans le brevet US 4,975,282.

Les liposomes sont le plus souvent obtenus à partir des MLV classiques décrits ci-dessus, après application d'un très fort cisaillement (par ultrasons ou presse de French), les transformant en vésicules unilamellaires ou paucilamellaires caractérisées par la présence d'un coeur aqueux.

Les vésicules multilamellaires à structure en oignon se présentent, quant à elles, sous forme d'un empilement régulier de bicouches concentriques allant du coeur même des vésicules jusqu'à leur périphérie.

### 3°. Leur nature thermodynamique

A l'intérieur des vésicules multilamellaires classiques, le nombre de feuillets multilamellaires, leur nombre de couches, le nombre de repliements et leur arrangement sont des caractéristiques qui dépendent du mode de préparation et en particulier de phénomènes cinétiques intervenant lors de la réhydratation du film lipidique. La structure interne des vésicules n'est pas uniforme au sein d'une vésicule (zones de faible courbure, zones de forte courbure, zones multilamellaires, zones continues). Une telle structure n'est donc pas à l'équilibre thermodynamique. De plus, chaque vésicule a une constitution différente, et il n'y a donc pas uniformité de structure sur tout l'échantillon.

Il a été décrit, en particulier dans la demande WO 96/31194 des liposomes de type classique paucilamellaire constitués de quelques couches entourant un coeur aqueux, au sein desquels se trouvent associés une molécule et son stabilisant.

Des compositions dans lesquelles un agent actif se trouve encapsulé au sein de vésicules multilamellaires à structure en oignon, constituées, de leur centre jusqu'à leur périphérie, d'une succession de couches lamellaires séparées par un milieu liquide se trouvent déjà décrites dans différents brevets référencés ci-dessous. Ces vésicules peuvent être obtenues par un procédé comprenant la préparation d'une phase lamellaire cristal-liquide et sa transformation par application d'un cisaillement. Un tel procédé est en particulier décrit dans le brevet WO 93/19735 issu du brevet français FR 2 689 418 ou WO 95/18601 introduits ici par référence.

Selon le brevet français FR 2 689 418, cette transformation peut être faite lors d'une étape de cisaillement homogène de la pâte cristal-liquide, ce qui conduit à des vésicules encore appelées micro-capsules de taille contrôlée. Toutefois, en jouant sur la formulation de la phase lamellaire cristal-liquide, en particulier sur la nature des tensioactifs entrant dans sa composition, la transformation de cette phase cristal-liquide en vésicules peut être obtenue par simple sollicitation mécanique, en particulier lors du mélange des constituants.

Différentes applications de ce type de vésicules ont été décrites par la demanderesse. On citera, en particulier, la demande internationale WO 95/19707 qui décrit des compositions odorantes dans lesquelles un produit odorant se trouve incorporé au sein de telles vésicules multilamellaires, l'effet d'une telle encapsulation étant d'augmenter la rémanence de l'odeur, du fait du ralentissement de l'évaporation. On citera également la demande française FR 2 735 658 qui décrit des compositions à usage alimentaire dans lesquelles un produit ou un additif à usage alimentaire se trouve inclus au sein de telles vésicules multilamellaires, avec pour effet essentiel d'obtenir un relargage contrôlé particulièrement avantageux du produit encapsulé, la présence de la vésicule multilamellaire permettant en outre de protéger, avant leur introduction dans les compositions, les molécules souvent fragiles qui se trouvent incorporées en son sein.

Toutefois, une telle composition, même si elle apporte dans certains cas un effet déjà sensible sur la stabilisation des molécules fragiles, peut s'avérer insuffisante pour des molécules particulièrement sensibles ou dont on cherche à obtenir une stabilisation particulièrement accrue à l'égard d'un environnement a priori défavorable.

Ainsi les inventeurs de la présente invention ont maintenant découvert que l'action de protection de molécules fragiles déjà observée dans le cas particulier de certains produits du domaine alimentaire, pouvait être considérablement accrue par incorporation au sein des vésicules multilamellaires à structure en oignon d'un agent destiné à stabiliser ces molécules fragiles. Une telle présentation du couple produit actif/agent stabilisant permet d'obtenir une stabilisation accrue, donc une durée de vie des produits incorporant l'agent actif accrue avec des concentrations nettement plus faibles en agent stabilisant, ce qui est un avantage considérable de la présente invention.

Cette invention s'avère intéressante dans tous les domaines où l'on cherche à protéger un agent actif vis-à-vis d'une action de dégradation. Elle s'applique ici aux enzymes.

L'invention fournit un moyen particulièrement efficace permettant d'assurer à la fois la fonction d'immobilisation d'une enzyme et sa protection vis-à-vis du milieu extérieur en vue d'améliorer sa stabilité.

Ainsi, la présente invention offre une solution particulièrement économique et efficace aux problèmes liés à la stabilisation des enzymes avec, en outre, tous les avantages liés à la technique d'encapsulation utilisée :
- grande souplesse de formulation,
- grande variété des tensioactifs utilisables,
- capacité de mettre en oeuvre simultanément plusieurs actifs,
- possibilité de recourir à plusieurs agents, en vue d'augmenter encore la stabilisation,
- stabilité accrue en milieu aqueux, aussi bien simple que complexe (gel, détergent, émulsion).

Ainsi, selon l'une de ses caractéristiques essentielles, l'invention concerne des compositions contenant au moins une enzyme et au moins un agent destiné à éviter la dégradation de ladite enzyme encapsulés au sein de vésicules multilamellaires sous forme d'un empilement régulier de bicouches concentriques comprenant au moins un agent tensioactif, lesdites bicouches allant du coeur même desdites vésicules jusqu'à leur périphérie et étant séparées par un liquide interstitiel.

Par structure en "oignon", on entend, comme exposé précédemment, une structure multilamellaire, dans laquelle les vésicules de forme sensiblement sphérique sont constituées d'une succession de bicouches concentriques et, cela, du centre à la périphérie des vésicules, d'où le nom de structure en oignon utilisé par analogie, pour qualifier de telles structures.

Ces structures peuvent être mises en évidence par examen microscopique des compositions. L'observation se fait en utilisant un microscope optique en lumière polarisée, dans lequel une phase lamellaire, biréfringente est visible. Elle se manifeste par une texture caractéristique, liée à la présence des défauts (joints de grains) entre les domaines de phase orientés différemment. Dans le cas de la phase concentrée de vésicules, la texture est caractérisée par son caractère uniforme et fin, relié à la taille des vésicules. Dans la phase dispersée de vésicules, celles-ci sont visibles sous la forme de points plus ou moins résolus (en fonction de la taille), légèrement biréfringents. La biréfringence ne s'observe que lorsque la dispersion n'est pas trop diluée. Il y aura donc lieu, si la dispersion est relativement diluée de procéder à une opération préalable de concentration pour mettre clairement en évidence la biréfringence caractéristique de la présence des vésicules à structure en oignon.

Le principe de l'invention consiste à utiliser les vésicules comme des micro-récipients contenant la molécule (enzyme) à protéger, et empêchant la réaction de dégradation de se produire. Pour cela, le rôle de la vésicule est double : d'une part isoler la molécule active de son environnement, et d'autre part apporter les additifs nécessaires à la stabilisation, ce qui s'avère particulièrement intéressant pour les molécules sensibles. L'un des avantages majeurs de la vésicule est de confiner la molécule fragile et sa protection dans un petit volume, beaucoup plus faible que le volume total de la préparation, et donc d'éviter ainsi l'effet de dilution, permettant de ce fait l'utilisation d'une plus faible quantité de molécule protectrice.

Selon une variante avantageuse de l'invention, les vésicules ont des dimensions comprises entre 0,1 et 50 µm, de préférence entre 0,2 et 10 µm.

De telles structures sont avantageusement obtenues par incorporation de l'enzyme et de l'agent destiné à le stabiliser dans une phase lamellaire cristal-liquide comprenant au moins un agent tensioactif puis transformation, par application d'un cisaillement, de cette phase cristal-liquide lamellaire en une phase dense de vésicules multilamellaires de petite taille.

Ce cisaillement pourra être un cisaillement homogène, ce qui présente l'avantage de conduire à des vésicules de taille parfaitement homogène. Toutefois, une simple agitation mécanique pourra s'avérer suffisante pour conduire à la formation des vésicules multilamellaires de l'invention.

Selon le brevet français FR-2 689 418, cette transformation peut être faite lors d'une étape de cisaillement homogène de la phase cristal-liquide, ce qui conduit à des vésicules ou microcapsules de taille contrôlée. Toutefois, en jouant sur la formulation de la phase lamellaire cristal-liquide, en particulier sur la nature des tensioactifs entrant dans sa composition, la transformation de cette phase cristal-liquide en vésicules peut être obtenue par simple sollicitation mécanique, en particulier lors du mélange des constituants.

Comme cela ressort en particulier des exemples illustratifs de l'invention, le choix des agents tensioactifs utilisables pour former les membranes des vésicules multilamellaires de l'invention est très large. Toutefois, on choisira ces agents tensioactifs en fonction du domaine d'utilisation de la composition visée. Dans de nombreux cas, l'application envisagée comporte des contraintes qui limitent le choix des tensioactifs. Il s'agit souvent de contraintes législatives ou liées à des normes. Ainsi, dans le domaine de la cosmétique, le catalogue de l'INCI (International Nomenclature of Cosmetic Ingrédients) fournit la liste des produits autorisés, dans le cas de l'agro-alimentaire on se réfère à la liste positive des additifs autorisés, ou dans celui de la pharmacie, à la pharmacopée.

La formulation fait avantageusement intervenir un mélange de molécules tensioactives. Il est généralement utilisé au moins deux tensioactifs différents ayant des balances hydrophile-lipophile différentes, ce qui permet de régler en continu les propriétés des bicouches et ainsi de contrôler l'apparition de l'instabilité qui gouverne la formation des vésicules multilamellaires.

Selon une variante particulièrement avantageuse de l'invention, on utilisera un mélange de deux agents tensioactifs dits respectivement agent tensioactif lipophile, présentant une balance hydrophile-lipophile (HLB) comprise entre 3 et 7, et agent tensioactif hydrophile, présentant une HLB comprise entre 8 et 15.

Selon une autre variante avantageuse de l'invention, les membranes des vésicules contiennent au moins un agent tensioactif polymère ou un polymère présentant des propriétés amphiphiles.

C'est le cas par exemple des poloxamères, et autres dérivés copolymères de l'oxyde d'éthylène et de l'oxyde de propylène éventuellement modifiés par adjonction de chaînes hydrophobes.

On peut citer à titre d'exemples non exhaustifs la famille des Pluronic^{®} et Lutrol^{®} (BASF), les hydroxystéararate de polyéthylène (Solutol^{®} de BASF ou MYRJ^{®} de ICI).

L'invention s'applique en particulier à des enzymes sensibles à des réactions plus spécifiques telles que l'autoprotéolyse dans le cas des protéases.

Un domaine où l'invention trouve des applications particulièrement intéressantes est celui de la cosmétique et de la dermatologie où de nombreux actifs sont connus pour leur fragilité.

Un autre domaine où l'invention trouve une application particulièrement intéressante est celui des médicaments pour lesquels les notions de fragilité de la molécule, et de limitation des additifs autorisés sont encore plus pertinentes que dans la cosmétique. -

L'agent destiné à stabiliser l'enzyme sera choisi en fonction de la nature de cette enzyme et du type de dégradation que l'on cherche à éviter et, cela, en tenant compte en outre du type d'application visée.

C'est le cas également lorsque le produit actif voit sa stabilité modifiée par une variation de pH susceptible, par exemple, de provoquer une réaction d'hydrolyse ou encore de modifier le potentiel redox du produit actif. On choisira alors d'encapsuler un agent stabilisateur permettant de modifier localement le pH, de façon à accroître la stabilité du produit actif.

Lorsque l'on souhaite améliorer la stabilité d'un produit actif sensible à l'oxydation, la vésicule contient avantageusement un produit connu pour ses propriétés anti-oxydantes du fait de ses propriétés réductrices ou du fait de son action pour diminuer le risque d'oxydation par effet séquestrant, par exemple par effet séquestrant des traces d'ions métalliques catalyseurs de l'oxydation contenus dans le milieu, ou par action sur le pH du milieu lorsque le potentiel redox dépend du pH.

Ainsi, on citera de façon non exhaustive à titre d'agents anti-oxydants :
l'acide ascorbique et ses dérivés,
l'acide citrique et ses dérivés (aussi utilisés comme séquestrant),
l'acide glutamique, les glutamates et leurs dérivés,
l'acide éthylène diamine tétraacétique (EDTA) (séquestrant),
l'acide lactique et ses dérivés (aussi utilisés pour ajuster le pH),
l'acide tartrique et ses dérivés (aussi utilisés pour ajuster le pH et comme agent séquestrant),
la benzophénone,
les bioflavonoïdes,
le butylhydroxy hydroxyanisol,
le butylhydroxy hydroxytoluène,
le carotène et ses dérivés,
le chlorobutanol,
les gallates de propyle, d'octyle ou de dodécyle,
le sulfite de sodium ou de potassium, et les composés apparentés tels que le bisulfite ou le pyrosulfite,
les tocophérols (alpha, delta ou gamma) et leurs dérivés,
d'une manière générale tous les additifs alimentaires des classes E3xx et E22x de la classification européenne des additifs alimentaires.

A titre d'exemple, la vitamine C et ses dérivés sont bien connus pour leur sensibilité à l'oxydation. On a clairement mis en évidence qu'on pouvait

L'agent destiné à stabiliser le produit actif peut également faire partie de la membrane de la vésicule, s'il a des propriétés amphiphiles.

Il existe des cas où l'agent stabilisant jouera également le rôle d'agent tensioactif participant à la formulation des membranes des vésicules. De tels exemples seront donnés plus loin à propos de la stabilisation des enzymes.

On utilisera comme agent stabilisant, d'une façon générale, soit un additif connu pour stabiliser ou protéger les protéines, désigné ci-après par additif protecteur non spécifique des enzymes, soit un agent spécifique destiné à stabiliser spécifiquement une enzyme.

Ainsi, dans le cas des protéases, un inhibiteur de protéases permettra d'éviter l'autoprotéolyse.

Selon une première variante, où l'on utilise un additif protecteur non spécifique, celui-ci est choisi avantageusement parmi les produits qui sont connus pour agir sur la conformation de l'enzyme. A titre d'exemple de tels produits, on citera en particulier des ions dont l'effet est d'augmenter la force ionique et de se fixer sur certains sites chargés de l'enzyme ou des molécules susceptibles d'engager des liaisons faibles avec la protéine.

L'homme du métier comprendra aisément que les ions les plus efficaces sont les ions relativement gros, par exemple des ions ammoniums et ammoniums quaternaires en ce qui concerne les cations et des sulfates, phosphates, carboxylates et polyacides carboxyliques en ce qui concerne les anions. L'homme du métier comprendra aisément que son choix pour obtenir la meilleure efficacité devra se porter sur des ions suffisamment gros ou attachés à une molécule suffisamment grosse.

Parmi les ions particulièrement intéressants pour stabiliser les enzymes, on citera l'ion calcium bien connu pour intervenir spécifiquement dans la réactivité de beaucoup d'enzymes, en particulier des protéases.

Dans la variante selon laquelle on utilise des molécules spécifiques stabilisatrices, on choisit avantageusement des molécules portant des fonctions susceptibles de se lier à l'enzyme, par exemple des molécules qui ont la possibilité de former des liaisons hydrogènes avec l'enzyme. Parmi ces molécules, on citera en particulier les alcools et les polyols, avantageusement des polyols associés à un dérivé du bore, en particulier à un ion borate, des amines éthoxylées et des oxydes d'amines. On pourra également choisir de recourir à des tensioactifs comportant plusieurs oxydes d'éthylène. De tels tensioactifs entreront dans la formulation des membranes des vésicules de l'invention et participeront à la stabilisation des enzymes.

Ainsi donc, on pourra citer à titre d'agents destinés à stabiliser des enzymes incorporées au sein de ces vésicules, des tensioactifs et des molécules amphiphiles contenant les fonctions suivantes ou substitués par les groupes suivants :
- ammoniums quaternaires,
- amines et éthanolamine,
- molécules portant une fonction phosphate, en particulier phospholipides,
- sels et esters d'acides gras,
- sels de polyacides,
- alcools,
- glycérol et ses esters (les glycérides),
- polyols (polyglycérides, polyéthylèneglycol, polypropylèneglycol),
- sucres (sorbitol, glucose, lactose, saccharose...)..

Les agents stabilisants des enzymes seront avantageusement choisis parmi les dérivés polymères qui contiennent des fonctions d'un des types précédents, en particulier :
◊ les polysaccharides modifiés ou non tels que :
   * agarose,
   * gommes guar,
   * carraghénanes,
   * acide alginique et alginate,
   * pectine,
   * chitosan,
◊ les polyvinylpyrrolidones, éventuellement substituées,
◊ les celluloses et dérivés de celluloses (alkylés ou fonctionnalisés),
◊ les polyacrylates,
◊ les polyvinylalcools (PVA) et les dérivés partiellement hydrolysés des polyvinylacétates,
◊ les polyacrylamides,
◊ les polyamides.

Les essais réalisés par les inventeurs de la présente invention montrent que la présence dans les vésicules multilamellaires incorporant une enzyme, en tant qu'agent destiné à éviter la dégradation de ladite enzyme, soit d'un tensioactif ou d'une molécule amphiphile présentant au moins une fonction azotée, soit d'un polymère présentant également une fonction azotée, permet d'améliorer considérablement la stabilisation de l'enzyme.

Lorsqu'on recourt comme agent de stabilisation d'une enzyme à un agent tensioactif comportant au moins une fonction azotée, on choisit de préférence un agent tensioactif, dans lequel la fonction azotée fait partie de la tête polaire dudit agent tensioactif.

Selon cette variante où les fonctions azotées font partie de la tête polaire du tensioactif, la queue hydrophobe est avantageusement constituée d'une
ou plusieurs chaînes carbonées. On peut donc définir indépendamment la nature de la partie hydrophobe qui ne varie pas beaucoup d'un tensioactif à l'autre : au moins une (deux en général) chaîne alkyle de 1 à 22 carbones, linéaire ou ramifiée, simple ou substituée, éventuellement portant un résidu cyclique ou aromatique, saturée ou portant une ou plusieurs insaturations, éventuellement substituée par d'autres fonctions. La ou les chaînes formant la partie hydrophobe de l'agent tensioactif peuvent être soit directement liées à l'atome d'azote de la fonction azotée, soit, le cas échéant, liées à l'un des substituants de l'azote.

Lorsqu'on recourt, pour stabiliser une enzyme, à l'utilisation d'un polymère portant au moins une fonction azotée, on le choisit avantageusement dans la liste suivante :
- polyacrylamides et produits de polymérisation ou de copolymérisation des dérivés de l'acrylamide,
- dérivés amidés de polysaccharides en particulier gommes guar quaternisées telles que le chlorure de guar hydroxypropyltrimonium.
- dérivés de la chitine tels que chitosan, sels de poly D-glucosamine, les contre-ions de ces polymères pouvant contenir une fonction amine ou dérivée (glutamate, par exemple),
- polyamides.

Dans le cas spécifique où l'on cherche à stabiliser une enzyme en évitant son autoprotéolyse, on co-encapsule avantageusement un inhibiteur de protéase, par exemple l'EDTA, le phénylméthanesulfonylfluorure, la 3,4-dichloroisocoumarine, la chymostatine.

Afin de renforcer le rôle de confinement joué par la vésicule, il peut être avantageux d'ajouter dans sa formulation un ou des polymères ou molécules à haut point de fusion, qui vont renforcer l'étanchéité de la vésicule. Ce renforcement de l'étanchéité peut aussi être obtenu par tout moyen permettant de diminuer l'échange avec le milieu de dispersion final, en particulier en enrobant la vésicule avec un polymère ou une cire, éventuellement une cire autoémulsifiable. Ainsi, l'invention couvre également selon une variante avantageuse des compositions dans lesquelles les vésicules comprennent en outre au moins un agent destiné à renforcer leur étanchéité, cet agent étant encapsulé au sein des vésicules ou constituant un enrobage externe de ces mêmes vésicules.

Selon une variante particulièrement avantageuse de l'invention, on pourra choisir comme agent destiné à stabiliser le produit actif un agent qui simultanément améliore l'étanchéité de la vésicule.

En résumé l'invention consiste à utiliser la microvésicule multilamellaire comme un milieu de confinement d'une enzyme et des additifs permettant sa stabilisation, éventuellement en renforçant son étanchéité.

Selon un autre de ses aspects, l'invention couvre également le procédé de préparation d'une composition telle que définie précédemment comprenant les étapes de :
- préparation d'une phase lamellaire cristal-liquide comprenant au moins un agent tensioactif et incorporant au moins une enzyme et un agent destiné à éviter la dégradation dudit agent actif,
- transformation de ladite phase cristal-liquide en vésicules multilamellaires à structure en oignon, par cisaillement ou sollicitation mécanique, en particulier par mélange des constituants de ladite phase lamellaire cristal-liquide.

Ce cisaillement sera avantageusement un cisaillement homogène tel qu'enseigné dans le brevet FR 2 689 418.

La transformation de la phase lamellaire cristal-liquide en vésicules multilamellaires à structure en oignon pourra être facilitée en jouant sur le choix tensioactifs. On pourra, en particulier, choisir comme indiqué précédemment un tensioactif présentant une haute HLB et un agent tensioactif présentant une basse HLB.

Par ailleurs, certains tensioactifs sont particulièrement adaptés pour favoriser cette transformation. Ainsi, c'est le cas par exemple des poloxamères, et autres dérivés copolymères de l'oxyde d'éthylène et de l'oxyde de propylène éventuellement modifiés par adjonction de chaînes hydrophobes. Ces composés sont particulièrement intéressants car ils apportent à la fois l'adaptation des propriétés élastiques de la membrane de tensioactifs et sa stabilisation par effet stérique. On peut citer à titre d'exemples non exhaustifs la famille des Pluronic^{®} et Lutrol^{®} (BASF), les hydroxystéararate de polyéthylène (Solutol^{®} de BASF ou MYRJ^{®} de ICI).

Enfin selon un dernier aspect, l'invention concerne également un procédé pour améliorer la stabilité d'un produit actif et éviter sa dégradation, caractérisé en ce qu'il consiste à encapsuler ledit produit actif au sein de vésicules multilamellaires telles que définies précédemment, présentant une structure en oignon et constituées, de leur périphérie jusqu'à leur centre, de membranes sous forme de bicouches concentriques comprenant au moins un agent tensioactif, lesdites membranes étant séparées par un liquide interstitiel, lesdites vésicules incorporant en leur sein au moins un agent destiné à éviter la dégradation dudit produit actif.

Pour les raisons exposées précédemment l'invention trouve une application particulièrement intéressante dans la stabilisation et/ou l'immobilisation des enzymes.

Ainsi, l'invention porte tout particulièrement sur un procédé pour protéger et/ou immobiliser une enzyme selon lequel on encapsule ladite enzyme au sein des vésicules multilamellaires à structure en oignon telles que définies précédemment, lesdites vésicules contenant en leur sein au moins un agent tel que défini précédemment destiné à éviter la dégradation de ladite enzyme.

Les excellents résultats obtenus en ce qui concerne la stabilisation d'une l'enzyme, lorsque celle-ci se trouve incorporée au sein d'une vésicule multilamellaire comprenant au moins un agent stabilisant choisi parmi les tensioactifs, les molécules amphiphiles et les polymères, portant au moins une fonction susceptible de se lier à ladite enzyme, ont semblé clairement liés à l'existence d'une liaison par laquelle l'enzyme se trouve en quelque sorte complexée à l'un des constituants des membranes de la vésicule. Ces résultats ont suggéré qu'une interaction du même type pourrait se produire entre la surface d'une vésicule multilamellaire incorporant dans ses membranes un tel agent stabilisant. Cette hypothèse a pu être confirmée par le fait qu'on a pu également constater un net effet de stabilisation d'une enzyme lorsque celle-ci se trouve mise en contact au sein d'une composition avec des vésicules multilamellaires à structure en oignon dont la formulation est telle que leurs membranes comprennent au moins un produit stabilisant choisi parmi les molécules amphiphiles, les tensioactifs et les polymères, portant au moins une fonction susceptible de se lier à ladite enzyme.

Ainsi donc, selon un autre aspect de la présente invention, elle concerne également un procédé pour protéger et/ou immobiliser une enzyme selon lequel on met ladite enzyme en présence de vésicules multilamellaires à structure en oignon, c'est-à-dire de vésicules constituées, de leur périphérie jusqu'à leur centre, de membranes sous forme de bicouches concentriques comprenant au moins un agent tensioactif, lesdites membranes étant séparées par un liquide interstitiel, lesdites vésicules incorporant en leur sein au moins un composé dit agent stabilisant, portant au moins une fonction susceptible de se lier à ladite enzyme. Cet agent stabilisant est choisi parmi les molécules amphiphiles, les tensioactifs et les polymères cités précédemment comme agent ayant un effet stabilisant sur une enzyme encapsulée.

Les exemples qui suivent donnés à titre purement illustratif de l'invention montrent comment il a été possible selon l'invention d'améliorer la stabilité de différents actifs réputés fragiles.

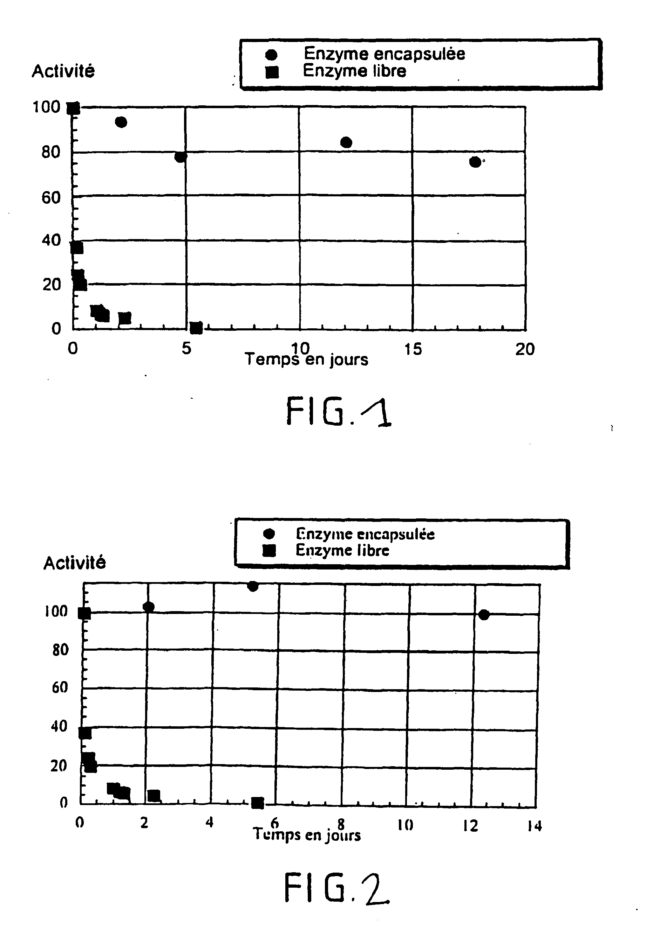

Ces exemples font également référence aux figures 1 et 2 :
- la figure 1, donnée en référence à l'exemple 1, illustre la stabilisation de la trypsine par encapsulation dans des vésicules multilamellaires à structure en oignon contenant, à titre d'agent stabilisant, un agent tensioactif portant une fonction ammonium ;
- la figure 2, donnée en référence à l'exemple 2, illustre la stabilisation de la trypsine par encapsulation dans une vésicule multilamellaire à structure en oignon incorporant un tensioactif de la famille des esterquats.

### EXEMPLES

Dans les exemples qui suivent, sauf indications contraires, les proportions sont données en pourcentages en poids.

### Exemple 1

### Stabilisation d'une enzyme par encapsulation dans une vésicule contenant un tensioactif portant une fonction ammonium

### 1) Principe du test de mise en évidence de la stabilisation des enzymes

Pour démontrer l'effet de stabilisation des enzymes, on mesure l'activité d'une enzyme encapsulée, en fonction du temps de vieillissement, par comparaison avec l'enzyme simplement mise en solution dans les mêmes conditions de vieillissement. L'enzyme encapsulée étant inaccessible, il faut préalablement la libérer pour pouvoir mesurer son activité. L'expérience comporte donc quatre étapes :
* préparation des échantillons
   * encapsulation de l'enzyme et dispersion des vésicules contenant l'enzyme
   * mise en solution à la même concentration de l'enzyme témoin
   * mise en vieillissement de la dispersion de vésicules et de la solution témoin
   * libération de l'enzyme des vésicules
   * mesure de l'activité enzymatique de l'échantillon testé et de l'échantillon témoin.

L'activité enzymatique est évaluée par une méthode classique de suivi de la réaction de dégradation d'un substrat caractéristique de l'enzyme.

### 2) Conditions de l'essai

### a) Enzyme

L'enzyme utilisée est la trypsine, c'est une sérine protéase (comme les protéases utilisées dans les détergents). Sa masse est de 23000 g/mol et sa taille est comprise entre 20 et 40 Å (entre 20 et 40.10⁻¹⁰ m).

Conditionnée sous forme lyophilisée, on la solubilise dans du tampon sous forme d'une solution à 4% en masse. C'est cette solution à 4% qui est utilisée dans les préparations.

Pour les mesures, les échantillons témoins sont préparés en prenant 50µl (50 mg) de cette solution pour 5 g de solution finale soit une teneur en enzyme dans la solution finale de 0.04% en masse.

### b)Vésicules

La teneur massique en enzyme dans les vésicules est de 0.15%. Sauf indication contraire, les vésicules sont préparées par mélange à température ambiante des tensioactifs puis incorporation de la solution aqueuse d'enzyme. On obtient ainsi une pâte visqueuse, de texture biréfringente caractéristique (observation au microscope optique en lumière polarisée). Cette pâte est dispersée par addition lente d'eau sous agitation à température ambiante.

Pour les mesures d'activité, toutes les dispersions de vésicules ont une teneur de 2% en vésicules. La teneur en enzyme de la dispersion de vésicules est donc de 0,04%.

### c) Tampon

C'est un tampon phosphate de composition :
NaCl : 8 g/l
KCl : 0.2g/l
Na₂HPO₄ : 1.15 g/l
KH₂PO₄ : 0.2 g/l

Le pH est compris entre 7.5 et 8 .

### d) Substrat

On utilise comme substrat le N-Benzoyl L arginyl ethyl ester (BAEE) qui est un substrat classiquement utilisé pour étudier l'activité de la trypsine dans la mesure où l'on sait que sa décomposition est catalysée par la trypsine. Le produit de réaction absorbe dans l'UV.

### e) Mesure d'activité

Elle est réalisée de la façon suivante :

### e1. Destruction des vésicules :

On prélève 25µl d'échantillon et on ajoute 20 µl de solution de triton X100 (Sigma) à 10%. On laisse agir environ 30 secondes.

### e2. Mesure de l'activité enzymatique:

On prélève 37µl de solution de substrat à 0,024 mol/l, on ajoute 950 µl de tampon et 22,5 µl d'échantillon. On suit la cinétique pendant 10 minutes à une longueur d'onde de 253 nm et à 20°C.

Le vieillissement des échantillons est effectué en étuve, à la température de 37°C.

### 3) Formulation testée

Agents tensioactifs utilisés :
Polysorbate 60 : Polyoxyéthylène (20) sorbitan monostéarate.
DODMAB : bromure de diméthyl dioctadécyl ammonium.

### Formulation :

- 15% solution aqueuse d'enzyme.
- 35% eau
- 25% DODMAB.
- 25% Polysorbate 60.

### Préparation :

Les tensioactifs et la solution d'enzyme sont mélangés grossièrement à température ambiante, puis le mélange est introduit dans une cellule de type Couette en procédant selon le brevet WO93/19735.

Le cisaillement est réalisé à 25 °C pendant 20 minutes, à un taux compris entre 50 et 100 s⁻¹ .

### 4) Résultats

La dégradation de l'enzyme est suivie par spectroscopie UV.

La figure 1 représente les variations de l'activité enzymatique au cours du temps pour l'enzyme encapsulée, en comparaison avec les variations observées pour l'enzyme libre. L'activité initiale est fixée arbitrairement à 100 pour le tracé de la courbe.

La figure 1 met clairement en évidence la stabilisation de l'enzyme du fait de son encapsulation.

### Exemple 2 : Stabilisation d'une enzyme par encapsulation dans une vésicule contenant un esterquat

On procède comme dans l'exemple 1. Le tensioactif utilisé dans cet exemple fait partie de la famille des esterquat. Il s'agit du : N ,N di(" acyl " oxy-2-éthyl), N-hydroxy-2-éthyl, N-méthyl amonium methosulfate en solution dans l'isopropanol commercialisé par CECA sous la marque Noxamium 920.
Formulation (en pourcentages en poids)

| | |
|---|---|
| solution aqueuse d'enzyme | 15% |
| eau | 35% |
| Noxamium 920 | 50% |

### Préparation

La préparation des vésicules est analogue à celle de l'exemple 1.

La figure 2 représente les variations de l'activité enzymatique au cours du temps pour l'enzyme encapsulée en comparaison avec les variations observées pour l'enzyme libre. L'activité initiale est arbitrairement fixé à 100.

Cette figure met clairement en évidence la stabilisation de l'enzyme du fait de son encapsulation.

## Revendications

1. Composition contenant au moins une enzyme et au moins un agent destiné à éviter la dégradation de ladite enzyme encapsulés au sein de vésicules multilamellaires sous forme d'un empilement régulier de bicouches concentriques comprenant au moins un agent tensioactif, lesdites bicouches allant du coeur même desdites vésicules jusqu'à leur périphérie et étant séparées par un liquide interstitiel.

2. Composition selon la revendication 1, **caractérisée en ce que** lesdites vésicules ont des dimensions comprises entre 0,1 et 50 µm, de préférence entre 0,2 et 10 µm.

3. Composition selon la revendication 1 ou 2, **caractérisée en ce que** les bicouches desdites vésicules comprennent un mélange de deux agents tensioactifs dits respectivement agent tensioactif lipophile, présentant une balance hydrophile-lipophile (HLB) comprise entre 3 et 7, et agent tensioactif hydrophile, présentant une HLB comprise entre 8 et 15.

4. Composition selon l'une des revendications 1 à 3, **caractérisée en ce que** les bicouches des vésicules contiennent au moins un agent tensioactif polymère ou un polymère présentant des propriétés amphiphiles.

5. Composition selon l'une des revendications 1 à 4, **caractérisée en ce que** ledit agent destiné à éviter la dégradation de ladite enzyme est un agent stabilisant connu pour stabiliser les protéines, en agissant sur la conformation de l'enzyme, en particulier un ion, par exemple un ion calcium.

6. Composition selon l'une des revendications 1 à 4, **caractérisée en ce que** ledit agent stabilisant est un agent portant des fonctions susceptibles de se lier à ladite enzyme.

7. Composition selon l'une des revendications 1 à 6, **caractérisée en ce que** ledit d'agent destiné à stabiliser ladite enzyme est choisi parmi les agents tensioactifs et les molécules amphiphiles contenant les fonctions suivantes ou substitués par les groupes suivants :
• ammoniums quaternaires,
• amines et éthanolamine,
• molécules portant une fonction phosphate, en particulier phospholipides,
• sels et esters d'acides gras,
• sels de polyacides,
• alcools,
• glycérol et ses esters (les glycérides),
• polyols, tels que polyglycérides, polyéthylèneglycol, polypropylèneglycol,
• sucres tels que sorbitol, glucose, lactose, saccharose.

8. Composition selon l'une des revendications 1 à 7, **caractérisée en ce que** ledit agent destiné à stabiliser ladite enzyme est un polymère, choisi dans le groupe constitué :
- des polysaccharides modifiés ou non, tels que l'agarose, les gommes guar, les carraghénanes, l'acide alginique et les alginates, la pectine, le chitosan,
- des polyvinylpyrrolidones éventuellement substituées,
- de la cellulose et des dérivés de cellulose tel que les dérivés alkylés ou fonctionnalisés,
- des polyacrylates,
- des polyvinylalcool (PVA) et des dérivés partiellement hydrolysés des polyvinylacétates,
- des polyacrylamides,
- des polyamides.

9. Composition selon l'une des revendications 1 à 8, **caractérisée en ce que** ledit agent destiné à éviter la dégradation de ladite enzyme est un composant comportant au moins une fonction azotée, en particulier un agent tensioactif ou un polymère.

10. Composition selon l'une des revendications 1 à 9, **caractérisée en ce que** ledit agent destiné à éviter la dégradation dudit agent actif présente un caractère amphiphile lui conférant un rôle actif dans la formulation des bicouches desdites vésicules.

11. Composition selon l'une des revendications 1 à 10, **caractérisée en ce que** lesdites vésicules comprennent en outre au moins un agent destiné à renforcer leur étanchéité, ledit agent étant encapsulé au sein desdites vésicules ou constituant un enrobage externe desdites vésicules.

12. Composition selon l'une quelconque des revendications 1 à 11,
**caractérisée en ce que** ladite enzyme est une protéase.

13. Procédé de préparation d'une composition selon l'une des revendications 1 à 12, **caractérisé en ce qu'**il comprend les étapes de :
- préparation d'une phase lamellaire cristal-liquide comprenant au moins un agent tensioactif et incorporant au moins une enzyme et un agent destiné à éviter la dégradation de ladite enzyme,
- transformation de ladite phase cristal-liquide en vésicules multilamellaires à structure en oignon, par cisaillement ou sollicitation mécanique, en particulier par mélange des constituants de ladite phase lamellaire cristal-liquide.

14. Procédé selon la revendication 13, **caractérisé en ce que** ledit cisaillement est un cisaillement homogène.

15. Procédé pour améliorer la stabilité d'une enzyme et éviter sa dégradation, **caractérisé en ce qu'**il consiste à encapsuler ladite enzyme au sein de vésicules multilamellaires telles que définies dans l'une des revendications 1 à 12 ou obtenues selon le procédé de l'une des revendications 13 ou 14, lesdites vésicules étant sous forme d'un empilement régulier de bicouches concentriques comprenant au moins un agent tensioactif, lesdites bicouches allant du coeur même desdites vésicules jusqu'à leur périphérie et étant séparées par un liquide interstitiel, lesdites vésicules incorporant en leur sein au moins un agent destiné à éviter la dégradation de ladite enzyme.

16. Procédé pour protéger et/ou immobiliser une enzyme **caractérisé en ce qu'**il consiste à mettre ladite enzyme en présence de vésicules multilamellaires sous forme d'un empilement régulier de bicouches concentriques comprenant au moins un agent tensioactif, lesdites bicouches allant du coeur même desdites vésicules jusqu'à leur périphérie et étant séparées par un liquide interstitiel, lesdites vésicules incorporant en leur sein au moins un agent destiné à éviter la dégradation de ladite enzyme tel que défini dans l'une des revendications 5 à 10 ou à encapsuler ladite enzyme au sein de vésicules multilamellaires telles que définies dans l'une des revendications 1 à 11 ou obtenues selon le procédé de l'une des revendications 14 ou 15, lesdites vésicules incorporant en leur sein au moins un agent destiné à éviter la dégradation de ladite enzyme tel que défini dans l'une des revendications 5 à 10.

17. Procédé selon la revendication 15 ou 16, **caractérisé en ce que** ladite enzyme est une protéase.

## Claims

1. Composition containing at least one enzyme and at least one agent intended to avoid the degradation of said enzyme encapsulated within multilamellar vesicles in the form of a regular stack of concentric bilayers comprising at least one surfactant, -said bilayers going from the very core of said vesicles to the periphery and being separated by an interstitial liquid.

2. Composition according to claim 1, **characterized in that** said vesicles are of dimensions lying in the range 0.1 µm to 50 µm, preferably in the range 0.2 µm to 10 µm.

3. Composition according to claim 1 or 2, **characterized in that** the bilayers of said vesicles comprise a mixture of two surfactants respectively referred to as a lipophilic surfactant, having a hydrophilic-lipophilic balance (HLB) in the range 3 to 7, and a hydrophilic surfactant, having an HLB in the range 8 to 15.

4. Composition according to one of claims 1 to 3, **characterized in that** the bilayers of the vesicles contain at least one polymer surfactant or a polymer having amphiphilic properties.

5. Composition according to one of claims 1 to 4, **characterized in that** said agent for avoiding degradation of said enzyme is a known stabilizing agent for stabilizing proteins, acting on the conformation of the enzyme, in particular an ion, e.g. a calcium ion.

6. Composition according to one of claims 1 to 4, **characterized in that** said agent for avoiding degradation of said enzyme is an agent carrying functions suitable for bonding to said enzyme.

7. Composition according to one of claims 1 to 6, **characterized in that** said agent for stabilizing said enzyme is selected from surfactants and amphiphilic molecules containing the following functions or substituted by the following groups:
• quaternary ammoniums;
• amines and ethanolamine;
• molecules carrying a phosphate function, in particular phospholipids;
• salts and esters of fatty acids;
• salts of polyacids;
• alcohols;
• glycerol and esters thereof (glycerides);
• polyols, such as polyglycerides, polyethyleneglycol, polypropyleneglycol; and
• sugars such as sorbitol, glucose, lactose, saccharose.

8. Composition according to one of claims 1 to 7, **characterized in that** said agent for stabilizing said enzyme is a polymer, selected from the group constituted by:
• optionally modified polysaccharides such as agarose, guar gums, carrageenans, alginic acid and alginates, pectin, chitosan;
• optionally substituted polyvinylpyrrolidones;
• cellulose and cellulose derivatives such as alkylated or functionalized derivatives;
• polyacrylates;
• polyvinylalcohol (PVA) and partially hydrolyzed derivatives of polyvinylacetates;
• polyacrylamides; and
• polyamides.

9. Composition according to one of claims 1 to 8, **characterized in that** said agent for avoiding degradation of said enzyme is a compound having at least one nitrogen-containing function, in particular a surfactant or a polymer.

10. Composition according to one of claims 1 to 9, **characterized in that** said agent for avoiding degradation of said active agent has an amphiphilic nature conferring to it an active role in the formulation of the bilayers of said vesicles.

11. Composition according to one of claims 1 to 10, **characterized in that** said vesicles furthermore comprise at least one agent for reinforcing their leakproofing, said agent being encapsulated within said vesicles or constituting an external coating of said vesicles.

12. Composition according to one of claims 1 to 11, **characterized in that** said enzyme is a protease.

13. Method of preparing a composition according to one of claims 1 to 12, the method being **characterized in that** it comprises the steps of:
• preparing a liquid crystal lamellar phase comprising at least one surfactant and incorporating at least one enzyme agent and an agent for avoiding degradation of said enzyme and
• transforming said liquid crystal phase into multilamellar vesicles of onion-structure by shear or by applying mechanical force thereto, in particular by mixing together the components of said liquid crystal lamellar phase.

14. Method according to claim 13, **characterized in that** said shear is homogeneous shear.

15. Method of improving the stability of an enzyme and of avoiding its degradation, the method being **characterized in that** it consists in encapsulating said enzyme within multilamellar vesicles as defined in one of claims 1 to 12 or as obtained by the method of claims 13 or 14, said vesicles being in the form of a regular stack of concentric bilayers comprising at least one surfactant, said bilayers going from the very core of said vesicles to the periphery and being separated by an interstitial liquid, said vesicles incorporating within them at least one agent for avoiding degradation of said enzyme.

16. Method of protecting and/or immobilizing an enzyme, the method being **characterized in that** it consists in putting said enzyme in the presence of multilamellar vesicles in the form of a regular stack of concentric bilayers comprising at least one surfactant, said bilayers going from the very core of said vesicles to the periphery and being separated by an interstitial liquid, said vesicles incorporating within them at least one agent for avoiding degradation of said enzyme as defined in any one of claims 5 to 10 or in encapsulating said enzyme within multilamellar vesicles as defined in any one of claims 1 to 11, or as obtained by the method of claim 14 or 15, said vesicles incorporating within them at least one agent for avoiding degradation of said enzyme as defined in any one of claims 5 to 10.

17. Method according to claim 15 or 16, **characterized in that** said enzyme is a protease.

## Patentansprüche

1. Zusammensetzung, die wenigstens ein Enzym sowie wenigstens eine Substanz, die dazu bestimmt ist, den Abbau des Enzyms zu verhindern, enthält, die im Inneren von multilamellaren Vesikeln in Form einer gleichmäßigen Aufschichtung von konzentrischen, wenigstens eine oberflächenaktive Substanz enthaltenden Doppelschichten eingekapselt sind, wobei die Doppelschichten vom Kern selbst der Vesikel bis zu deren Umfang reichen und durch eine interstitielle Flüssigkeit getrennt sind.

2. Zusammensetzung nach Anspruch 1, **dadurch gekennzeichnet, daß** die Vesikel Abmessungen zwischen 0,1 und 50 µm, vorzugsweise zwischen 0,2 und 10 µm aufweisen.

3. Zusammensetzung nach Anspruch 1 oder 2, **dadurch gekennzeichnet, daß** die Doppelschichten der Vesikel eine Mischung aus zwei oberflächenaktiven Substanzen enthalten, der sogenannten lipophilen oberflächenaktiven Substanz mit einer Hydrophil-Lipophil-Balance (HLB) zwischen 3 und 7 bzw. der sogenannten hydrophilen oberflächenaktiven Substanz mit einer HLB zwischen 8 und 15.

4. Zusammensetzung nach einem der Ansprüche 1 bis 3, **dadurch gekennzeichnet, daß** die Doppelschichten der Vesikel wenigstens eine polymere oberflächenaktive Substanz oder ein Polymer mit amphiphilen Eigenschaften enthalten.

5. Zusammensetzung nach einem der Ansprüche 1 bis 4, **dadurch gekennzeichnet, daß** die Substanz, die dazu bestimmt ist, den Abbau des Enzyms zu verhindern, ein Stabilisator ist, welcher dafür bekannt ist, Proteine durch Einwirken auf die Konformation des Enzyms zu stabilisieren, insbesondere ein Ion, beispielsweise ein Kalziumion.

6. Zusammensetzung nach einem der Ansprüche 1 bis 4, **dadurch gekennzeichnet, daß** der Stabilisator eine Substanz ist, die Gruppen trägt, welche geeignet sind, mit dem Enzym eine Verbindung einzugehen.

7. Zusammensetzung nach einem der Ansprüche 1 bis 6, **dadurch gekennzeichnet, daß** die Substanz, welche dazu bestimmt ist, das Enzym zu stabilisieren, aus den oberflächenaktiven Substanzen und den amphiphilen Molekülen ausgewählt ist, welche die folgenden Gruppen enthalten oder durch die folgenden Gruppen substituiert sind:
- quartäre Ammoniumverbindungen,
- Amine und Ethanolamin,
- Moleküle, die eine Phosphatgruppe tragen, insbesondere Phospholipide,
- Salze und Ester von Fettsäuren,
- Salze von Polysäuren,
- Alkohole,
- Glycerin und seine Ester (Glyceride)
- Polyole, wie Polyglyceride, Polyethylenglykol, Polypropylenglykol,
- Zucker, wie Sorbitol, Glukose, Laktose, Saccharose.

8. Zusammensetzung nach einem der Ansprüche 1 bis 7, **dadurch gekennzeichnet, daß** die Substanz, welche dazu bestimmt ist, das Enzym zu stabilisieren, ein Polymer ist, das aus der Gruppe bestehend aus:
- modifizierten oder nicht modifizierten Polysacchariden, wie Agarose, Guargummi, Carrageenane, Alginsäure und Alginate, Pektin, Chitosan,
- Polyvinylpyrrolidonen, eventuell substituiert,
- Zellulose und Zellulosederivaten, wie alkylierte oder funktionalisierte Derivate,
- Polyacrylaten,
- Polyvinylalkohol (PVA) sowie partiell hydrolysierten Derivaten der Polyvinylacetate,
- Polyacrylamiden,
- Polyamiden
ausgewählt ist.

9. Zusammensetzung nach einem der Ansprüche 1 bis 8, **dadurch gekennzeichnet, daß** die Substanz, welche dazu bestimmt ist, den Abbau des Enzyms zu verhindern, eine Komponente mit wenigstens einer Stickstoffgruppe, insbesondere eine oberflächenaktive Substanz oder ein Polymer ist.

10. Zusammensetzung nach einem der Ansprüche 1 bis 9, **dadurch gekennzeichnet, daß** die Substanz, welche dazu bestimmt ist, den Abbau des Wirkstoffs zu verhindern, einen amphiphilen Charakter aufweist, der ihr bei der Formulierung der Doppelschichten der Vesikel eine aktive Rolle verleiht.

11. Zusammensetzung nach einem der Ansprüche 1 bis 10, **dadurch gekennzeichnet, daß** die Vesikel ferner wenigstens eine Substanz enthalten, die dazu bestimmt ist, deren Dichtigkeit zu verstärken, wobei die Substanz im Inneren der Vesikel eingekapselt ist oder eine Außenummantelung der Vesikel bildet.

12. Zusammensetzung nach einem der Ansprüche 1 bis 11, **dadurch gekennzeichnet, daß** das Enzym eine Protease ist.

13. Verfahren zur Herstellung einer Zusammensetzung nach einem der Ansprüche 1 bis 12, **dadurch gekennzeichnet, daß** es die folgenden Schritte umfaßt:
- Herstellen einer lamellaren Flüssigkristallphase, die wenigstens eine oberflächenaktive Substanz umfaßt und wenigstens ein Enzym sowie eine Substanz, die dazu bestimmt ist, den Abbau des Enzyms zu verhindern, enthält,
- Umwandlung der Flüssigkristallphase in multilamellare Vesikel mit Zwiebelstruktur, durch Scheren oder mechanische Beanspruchung, insbesondere durch Mischen der Bestandteile der lamellaren Flüssigkristallphase.

14. Verfahren nach Anspruch 13, **dadurch gekennzeichnet, daß** das Scheren ein homogenes Scheren ist.

15. Verfahren zur Verbesserung der Haltbarkeit eines Enzyms und zur Verhinderung seines Abbaus, **dadurch gekennzeichnet, daß** es darin besteht, das Enzym im Inneren von multilamellaren Vesikeln, wie sie in einem der Ansprüche 1 bis 12 definiert sind oder nach dem Verfahren aus einem der Ansprüche 13 oder 14 erhalten werden, einzukapseln, wobei die Vesikel in Form einer gleichmäßigen Aufschichtung von konzentrischen, wenigstens eine oberflächenaktive Substanz enthaltenden Doppelschichten vorliegen, wobei die Doppelschichten vom Kern selbst der Vesikel bis zu deren Umfang reichen und durch eine interstitielle Flüssigkeit getrennt sind, wobei die Vesikel in ihrem Inneren wenigstens eine Substanz enthalten, die dazu bestimmt ist, den Abbau des Enzyms.

16. Verfahren zum Schutz und/oder Festlegen eines Enzyms, **dadurch gekennzeichnet, daß** es darin besteht, das Enzym mit multilamellaren Vesikeln in Form einer gleichmäßigen Aufschichtung von konzentrischen, wenigstens eine oberflächenaktive Substanz enthaltenden Doppelschichten in Kontakt zu bringen, wobei die Doppelschichten vom Kern selbst der Vesikel bis zu deren Umfang reichen und durch eine interstitielle Flüssigkeit getrennt sind, wobei die Vesikel in ihrem Inneren wenigstens eine Substanz enthalten, die dazu bestimmt ist, den Abbau des Enzyms zu verhindern, wie sie in einem der Ansprüche 5 bis 10 definiert ist, oder das Enzym im Inneren von multilamellaren Vesikeln einzukapsein, wie sie in einem der Ansprüche 1 bis 11 definiert sind oder nach dem Verfahren aus einem der Ansprüche 14 oder 15 erhalten werden, wobei die Vesikel in ihrem Inneren wenigstens eine Substanz enthalten, die dazu bestimmt ist, den Abbau des Enzyms zu verhindern, wie sie in einem der Ansprüche 5 bis 10 definiert ist.

17. Verfahren nach Anspruch 15 oder 16, **dadurch gekennzeichnet, daß** das Enzym eine Protease ist.
